Europäisches Patentamt

European Patent Office

Office européen des brevets

(19)

(11) Publication number: **0 132 344**
A2

# (12) EUROPEAN PATENT APPLICATION

(21) Application number: **84304705.1**

(22) Date of filing: **10.07.84**

(51) Int. Cl.⁴: **A 61 B 5/02**, A 61 M 25/00

(30) Priority: **20.07.83 US 515661**

(43) Date of publication of application: **30.01.85**
Bulletin 85/5

(84) Designated Contracting States: **AT BE CH DE FR GB IT LI LU NL SE**

(71) Applicant: **PURDUE RESEARCH FOUNDATION, Hovde Hall Purdue University, West Lafayette Indiana 47907 (US)**

(72) Inventor: **Geddes, Leslie A., 400 N. River Road Apt. 1724, West Lafayette Indiana 47906 (US)**
Inventor: **Bourland, Joe D., 606 Wilshire Avenue, West Lafayette Indiana 47906 (US)**
Inventor: **Voorhees, William D., 110 Sarasota Drive, Indiana 47905 (US)**

(74) Representative: **Bannerman, David Gardner et al, Withers & Rogers 4 Dyer's Buildings Holborn, London, EC1N 2JT (GB)**

(54) Improved catheter based cardiac output sensor.

(57) A flexible catheter which is useful for positioning in a blood vessel an electrical sensor used to measure cardiac output. The catheter has a sinuously contoured portion exhibiting shape memory properties which permit it to be transluminally inserted into a blood vessel in a straightened position and released into the normal configuration after positioning. The sensor is positioned relative to the contoured portion such that after positioning the contoured portion prevents the sensor from approaching the blood vessel wall, thereby eliminating artifacts in the output signal from the sensor.

-1-

## IMPROVED CATHETER-BASED
## CARDIAC OUTPUT SENSOR

### Background of the Invention

The present invention relates generally to the field of catheters and, more specifically, to arteriovenous catheters which are equipped with a sensing device which permits measurement of cardiac output.

Certain methods for measuring cardiac output involve the catheterization of a blood vessel and transluminal insertion of a sensing device which is disposed on the catheter. The most common of these methods involves thermal dilution by injecting a cold sterile fluid into the bloodstream and sensing of the resulting change in blood temperature employing a thermister as the sensing means, thereby permitting calculation of cardiac output. Another more improved method, and for which one of the co-inventors herein is also co-inventor, involves the utilization of an indicator, such as saline, which alters blood resistivity, and an electrically calibrated conductivity sensor positioned at the tip of a catheter. The conductivity sensor is inserted for current injection to develop a potential which is proportional to blood resistivity for inscribing the dilution curve occurring due to injection of the indicator, which curve is utilized to determine cardiac output. This method is fully described in U.S. Patent Application Serial No. 370,568 to Geddes. et al.

In addition, the following patent references are believed to disclose other various devices relating to blood flow measurement, and, except for the Rodler and Brown references, relate specifically to devices for cardiac output measurement:

| Patent No. | Inventor | Issued |
|---|---|---|
| 3,433,935 | Sherman | 3/18/69 |
| 3,131,689 | Rodler | 5/5/64 |
| 4,329,993 | Lieber et al. | 5/18/82 |
| 4,236,527 | Newbower et al. | 12/2/80 |
| 3,733,899 | Auphan et al. | 5/22/73 |
| 3,678,922 | Phillips et al. | 7/25/72 |
| 3,651,318 | Czekajewski | 3/21/72 |
| 3,835,839 | Brown | 9/17/74 |

U. S. Patent No. 3,433,935 to Sherman is relevant for its description of a saline injection method for measuring cardiac output. This reference also discloses the use of either a standard catheter to withdraw a blood sample for measurement of blood conductivity, or a probe type catheter which allows the measurements to be made within the blood vessel.

U. S. Patent No. 3,131,689 to Rodler also discusses the measurement changes in the conductance or resistance of blood as a method for determining blood flow characteristics. The apparatus disclosed in Rodler impresses a voltage upon the entire body by use of primary electrodes, with secondary electrodes being used to measure changes in the blood.

U. S. Patent No. 4,329,993 to Lieber et al. and U. S. Patent No. 4,236.527 to Newbower et al. both disclose the use of a boone catheter which is inserted through the heart and into the pulmonary artery. Further, thermisters are utilized to determine changes in the thermal energy or characteristics of the blood as the result of an injected material such as saline. U. S. Patent No. 3,733,899 to Auphan et al. and U. S. Patent No. 3,678,922 to Phillips et al. also disclose thermal measurements for determining cardiac output. U. S. Patent No. 3,651,318 to Czekajewski discloses a cardiac output computer wherein an indicator is injected into the body blood stream and circulation of the indicator substance is evaluated to obtain cardiac output information. Lastly, U. S. Patent No. 3,835,839 to Brown is cited for its disclosure of an impedance plethysmograph.

It is noted that while catheterization presents a relatively safe and simple method for inserting cardiac output measuring devices, there are attendant disadvantages. Thus, it has been observed that artifacts occur in the signal sent from the cardiac output measurement sensing device which are not related to cardiac output. Obviously, the presence of any artifacts distort the output signal and provide inaccurate cardiac output data. The inventors herein have determined that these artifacts are primarily due to the sensor coming into contact or close contact with the blood vessel wall. The present invention eliminates these artifacts by providing a novel catheter arrangement which permits the sensing device to be positioned so that it will not approach the blood vessel wall, yet which also does not make more difficult the catheterization of the blood vessel.

## Summary of the Invention

One embodiment of the present invention is characterized by an apparatus for enabling cardiac output measurement by catheterization of a blood vessel. The apparatus comprises a sensing means for sensing a body parameter related to cardiac output and a flexible catheter providing a base for said sensing means. The catheter is characterized by having a contoured portion having a sinuous shape. The contoured portion exhibits shape memory properties which permit it to be straightened for transluminal insertion into a blood vessel and to resume the sinuously contoured shape after positioning within the blood vessel. The sensing means is arranged and disposed relative to the contoured portion in such fashion that the sensing means is prevented from approaching the blood vessel wall.

Accordingly, it is an object of the present invention to provide an improved catheter for a catheter-based cardiac output sensor.

Related objects and advantages of the present invention will be made apparent in the following figures and detailed description.

## Brief Description of the Drawings

FIGS. 1-6 are enlarged fragmentary views of respective alternative preferred embodiments of the catheter-based cardiac output sensor of the present invention, illustrating several different catheter configurations and sensor positions.

FIG. 7 is a diagrammatic fragmentary section view illustrating the problem which occurs when a standard-type catheter is inserted in a blood vessel for cardiac output measurement purposes.

FIG. 8 is a diagrammatic fragmentary section view illustrating the manner in which a catheter formed according to one embodiment of the present invention solves the problem depicted in FIG. 7.

## Description of the Preferred Embodiment

For the purposes of promoting an understanding of the principles of the invention, reference will now be made to the embodiment illustrated in the drawings and specific language will be used to describe the same. It will nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

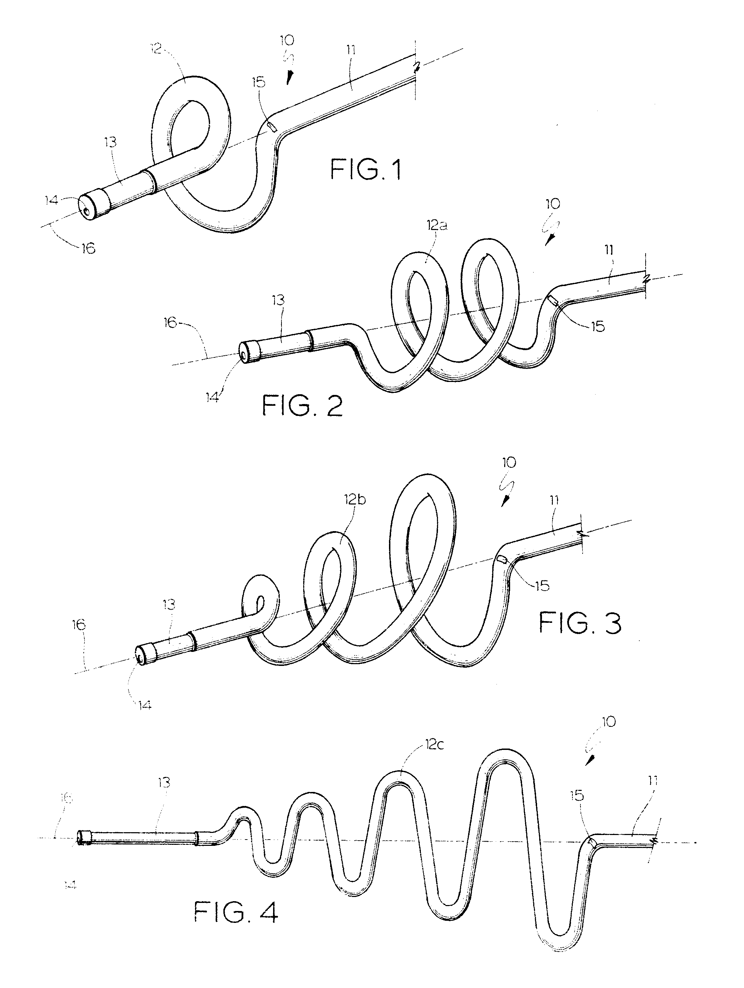

Referring now to the drawings, FIG. 1 depicts a preferred embodiment of the apparatus of the invention generally designated at 10. There is provided a flexible catheter 11, which is suitably sized and formed for transluminal insertion into a body blood vessel. Catheter 11 includes a contoured portion 12, which is positioned adjacent to and forward of a cardiac output sensor 15. As shown in FIG. 1, contoured portion 12 is seen to have a generally corkscrew shape formed about central axis 16, on which is positioned cardiac output sensor 15. Cardiac output sensor 15 is mounted on catheter 11 adjacent the proximal end of contoured portion 12. Contoured portion 12 has a maximum diameter d which is on the order of several times the diameter of catheter 11. The exact dimensions of contoured portion 12 will of course vary depending upon the size of the blood vessel in which it is to be inserted. However, it may be noted that dimension d is preferably much less than the diameter of the blood vessel so as to allow for variations in the size of the blood vessel passageway and provide minimum restriction thereof. Also, while FIG. 1 shows contoured portion 12 having only one convolution, it may be appreciated that contoured portion 12 may, alternatively have more than one convolution, such as is disclosed for contoured portions 12a and 12b depicted in FIGS. 2-3, respectively.

It is to be appreciated that contoured portion 12 of catheter 11 is hollow and flexible so that a stylet can be inserted therein, thereby straightening out contoured portion 12 for easy insertion of apparatus 10 within a body blood vessel. When the stylet, which may be of any conventionally known and suitable type, is removed from catheter 11, contoured portion 12 will immediately resume its contoured shape, thus exhibiting shape memory properties.

A balloon 13 and a distal port 14 are provided on catheter 11 forward of contoured portion 12. It is to be understood that balloon 13 provides flow guidance of catheter 11 when employed in accordance with well known medical techniques so that catheter 11 may be properly positioned within the pulmonary artery. Distal port 14 may be used for blood pressure measurement, sampling, infusion or injection purposes in a manner which is well known and understood by those familiar with the art.

It is to be noted that cardiac output sensor 15 has been diagrammatically shown for the reason that it may be any type of sensing element which is presently known and used for cardiac output measurement purposes, and which exhibits artifactual changes when the blood vessel wall is approached. Thus, for example, sensor 15 may be a conductivity cell, such as is fully disclosed in patent application USSN 370,568 to Geddes, et al. For further example, sensor 15 may also be a thermister, such as is used in practicing the well known thermal dilution method for measuring cardiac output previously mentioned. A yet further technique could involve using an optical sensing element such as an optical fiber in which light would be passed through in to measure $PO_2$, $PCO_2$ or a dye injected into the bloodstream. At this point it is important to understand that the catheter of the present invention, such as will be herein described, provides a major benefit when used with any type of cardiac output sensor which is transluminally inserted within a blood vessel.

Referring now to FIGS. 2-6, several alternative preferred embodiments of the apparatus of the present invention are depicted. It is to be understood that where elements in each of the alternative preferred embodiments correspond to a similar element described in the embodiment of FIG. 1, the same reference numerals are used. Thus, FIGS. 2-5 illustrate several alternative shapes for contoured portion 12 of FIG. 1. The construction of contoured portions 12a-d as depicted in each of the alternative preferred embodiments of FIGS. 2-5, respectively, is seen to be a generally sinuous shape having a maximum cross sectional dimension d which corresponds to that described for contoured portion 12 in FIG. 1. FIGS. 2 and 3 show contoured portions 12a and 12b, respectively, having a generally cork screw shape about central axis 16, which in FIG. 2 has two convolutions and in FIG. 3 includes three convolutions. Also, as shown in FIG. 3, the diameter of each of the convolutions may be progressively increased from the distal to the proximal end of contoured portion 12b, so as to give contoured portion 12b a tapered shape.

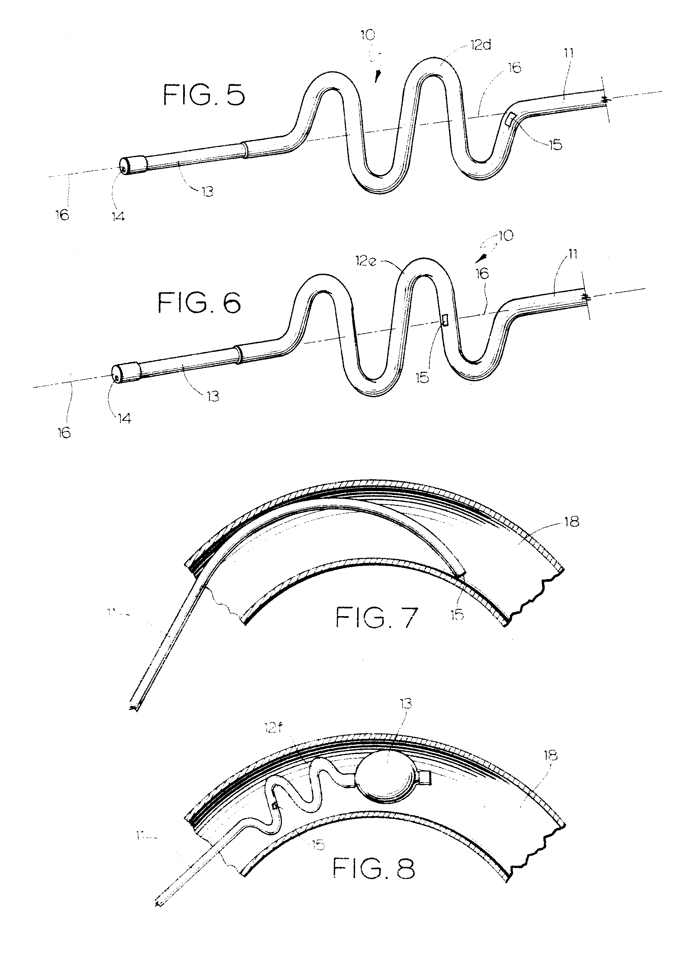

Referring now to FIGS. 4 and 5, it is observed that contoured portions 12c and 12d have a zig-zag or serpentine-like shape which is characterized by one or more S-shaped bends. FIG. 4 shows the contoured portion 12c having a configuration wherein the lateral width of each S-shaped bend increases in a direction away from the distal end of catheter 11, providing a taper which is analogous to that shown in FIG. 3.

It should be apparent from FIGS. 2-5 that sensor 15 is positioned in a location on catheter 11 identical to that described for FIG. 1. Alternatively, if the serpentine-like configuration shown in FIGS. 4 and 5 is employed, sensor 15 may be positioned on central axis 16 in an intermediate location along the contoured portion, as depicted in FIG. 6. Further, it may be noted that while it is possible to position sensor 15 laterally of central axis 16 and obtain some of the benefits of the present invention, such positioning will produce suboptimal results.

FIG. 7 illustrates the problems which occur when a standard catheter is used to position sensor 15 within the pulmonary artery in order to measure cardiac output. Thus, FIG. 7 shows that when catheter 11 is advanced transluminally within a blood vessel 18, sensor 15 may become positioned close to the vessel wall. Moreover, with the beating of the heart, and consequent pulsitile blood flow, the distal tip of catheter 11 housing sensor 15 will approach and recede from the vessel wall with each heart beat, causing large artifactual changes in conductivity. If sensor 15 is a conductivity cell used to sense changes in blood conductivity, it can be appreciated that any contact with the vessel wall causes inaccuracy to occur in the measurement of cardiac output. Moreover, it can readily be perceived that even if sensor 15 is located a certain distance behind the distal tip of catheter 11, it is still susceptible to coming into contact or approaching the blood vessel wall, thus causing artifacts to occur.

In FIG. 8, a catheter having a contoured portion similar to that shown in FIG. 6 is shown within a body blood vessel 18. It should be readily apparent from FIG. 8 that by using a catheter having a contoured portion 12d, it is possible to keep sensor 15 from ever approaching the blood vessel wall. It should also be noted that the contoured portions described in FIGS. 1-5 will perform similarly to prevent sensor 15 from becoming positioned too close to the blood vessel wall. In this manner, the presence of any artifacts associated with the contact of sensor 15 with the blood vessel wall is totally obviated.

It has been determined that artifacts are also caused if sensor 15 is a thermister, such as used when a thermal dilution method is employed to measure cardiac output. Thus, the use of a catheter-based sensor constructed in accordance with the teachings of the present invention will also improve the accuracy of thermal dilution measurement techniques.

While the invention has been illustrated and described in detail in the drawings and foregoing description, the same is to be considered as illustrative and not restrictive in character, it being understood that only the preferred embodiment has been shown and described and that all changes and modifications that come within the spirit of the invention are desired to be protected.

CLAIMS:

1.    A catheter-based cardiac output sensor, comprising:

a sensing means for measuring a body parameter related to cardiac output; and

a flexible catheter associated with said sensing means for said positioning said sensing means in the flow path of a blood vessel, said catheter including a contoured portion having a plurality of sinuous bends, said contoured position exhibiting shape memory properties which permit said contoured portion to be straightened during transluminal insertion into a blood vessel and to resume the sinuously contoured shape after positioning within said blood vessel, said sensing means arranged and disposed relative to said contoured portion in such fashion that said sensing means is prevented from approaching the wall of said blood vessel.

2.    The apparatus of claim 1 wherein said sinuous bends of said contoured portion of said catheter are characterized by one of a corkscrew and serpentine pattern.

3.    The apparatus of claim 2 wherein said catheter has proximal and distal ends and said contoured portion is positioned adjacent said distal end.

4.    The apparatus of claim 3 wherein the diameter of said contoured pattern increases from said distal end to said proximal end.

5.    The apparatus of claim 4 wherein said catheter further includes a balloon positioned between said contoured portion and said distal end, said balloon serving to provide flow guidance.

6.    The apparatus of claim 5 wherein said sensing means is positioned one of within or adjacent to said contoured portion.

7.    The apparatus of claim 6 wherein said sensing means is one of a conductivity cell, an optical sensing element and a thermister.

0132344

FIG. 1

FIG. 2

FIG. 3

FIG. 4

0132344

FIG. 5

FIG. 6

FIG. 7

FIG. 8